# EUROPEAN PATENT SPECIFICATION

(11) **EP 2 127 668 B1**
(45) Date of publication and mention of the grant of the patent: **17.08.2011**
(21) Application number: 08703523.4
(22) Date of filing: 21.01.2008
(51) Int. Cl.: A61K 38/45, A23L 1/305, A61P 1/00, A61P 29/00, C12N 9/98

(54) **Glycosyltransferase for treating Inflammatory Bowel Disease**
Glycosyltransferase zur Behandlung von entzündlichen Darmkrankheiten
Glycosyltransférase pour le traitement des maladies intestinales inflammatoires

(30) Priority: 26.01.2007 JP 2007016892
(43) Date of publication of application: 02.12.2009
(73) Proprietor: Amano Enzyme Inc., Nagoya-shi Aichi 460-0003 (JP); Joh, Takashi, Aichi 467-0046 (JP); Sasaki, Makoto, Aichi 463-0035 (JP)
(72) Inventor: JOH, Takashi, Nagoya-shi Aichi 467-0046 (JP); SASAKI, Makoto, Nagoya-shi Aichi 463-0035 (JP); KOIKEDA, Satoshi, Kakamigahara-shi Gifu 509-0108 (JP)
(74) Representative: Steinecke, Peter
(86) International application number: PCT/JP2008/050673
(87) International publication number: WO 2008/090831

(56) References cited:
- EP-A1- 0 457 919
- EP-A2- 0 968 719
- JP-A- 03 183 457
- JP-A- 2000 325 045
- LINDSAY J O ET AL: "Clinical, microbiological, and immunological effects of fructo-oligosaccharide in patients with Crohn's disease." March 2006 (2006-03), GUT MAR 2006, VOL. 55, NR. 3, PAGE(S) 348 - 355 , XP002575362 ISSN: 0017-5749 * abstract *
- DADDAOUA A. ET AL.: 'Goat milk oligosaccharides are anti-inflammatory in rats with hapten-induced colitis' JOURNAL OF NUTRITION vol. 136, no. 3, 2006, pages 672 - 676, XP008110892
- CHERBUT C. ET AL.: 'The prebiotic characteristics of fructooligosaccharides are necessary for reduction of TNBS-induced colitis in rats' JOURNAL OF NUTRITION vol. 133, no. 1, 2003, pages 21 - 27, XP008110893
- RUMI G. ET AL.: 'Protective effect of lactulose on dextran sulfate sodium-induced colonic inflammation in rats' DIGESTIVE DISEASES AND SCIENCES vol. 49, no. 9, 2004, pages 1466 - 1472, XP008110897
- WINKLER J. ET AL.: 'Fructo-oligosaccharide reduces inflammation in a dextran sodium sulphate mouse model of colitis' DIGESTIVE DISEASES AND SCIENCES, [Online] vol. 52, no. 1, 14 December 2006, pages 52 - 58, XP019464741 Retrieved from the Internet: <URL:http://www.springerlink.com/content/f1 3046123m150w6u/fulltext.pdf>

## Description

### TECHNICAL FIELD

The present invention relates to an enzyme composition comprising glucosyltransferase for use in the teatment or prevention of an inflammatory bowel disease.

### BACKGROUND ART

An inflammatory bowel disease (IBD) is inflammatory disease of the digestive tracts (mainly, small intestine and large intestine) from an unknown cause, and specifically includes ulcerative colitis (UC) and Crohn's disease (CD). UC is localized in the large intestine, and the characteristic lesion is a superficial ulcer and inflammation of the mucosa. The lesion is seen continuously in the ascending direction from the rectum. The symptoms include diarrhea, mucous and bloody stool, abdominal pain, fever, and the like. Meanwhile, CD causes discontinuous deep longitudinal ulcer with cobble stone appearance and full-thickness inflammation in all digestive tracts, mainly in the small intestine and large intestine, and is characterized by the formation of noncaseating granuloma. The main symptoms include abdominal pain with intestinal inflammation, diarrhea, fever, weight loss, and the like. In Japan, the number of patients with IBD is smaller, which is 10 - 20%, as compared with the number in Western countries. Recently, however, it has been a problem that the number of the patients has been increasing by about 10% every year. One of the causes thereof is thought to be a growing trend toward westernization of dietary habits including, for example, high-fat diet, low dietary fiber, high intake of sugar, and the like.

At present, the treatment method for IBD has not been established and many studies are being made. It has been revealed that intestinal bacteria are involved in intestinal inflammation of IBD. It is thought that the state of intestinal bacterial flora is closely related to the condition of IBD. In some research reports in which probiotics such as lactic acid bacteria are given to IBD model animals, improvement of some symptoms of IBD is observed. Therefore, the increase in lactic acid bacteria in the intestinal bacterial flora may improve the symptoms of IBD. In order to improve an intestinal bacterial flora, in addition to a probiotic preparation of orally administrating lactic acid bacteria, a method of orally administrating a component called prebiotic that promotes the proliferation of lactic acid bacteria is being considered. Although there are few reports about prebiotics, in an experiment using IBD model animals, it is shown that lactulose has an effect of reducing symptoms (see, non-patent document 1). Furthermore, since there also are few studies about reduction of symptoms of IBD by oligosaccharide that is well-known as a prebiotic, the efficacy of oral administration of oligosaccharide in reducing the symptoms of IBD has not been clarified.

By the way, oral administration of probiotic preparation or prebiotic preparation has the following problems. In the case of the probiotic preparation, since the survival rate of probiotic preparation in the human intestine is different depending upon the kinds of bacteria to be orally administrated, it is necessary to select kinds of bacteria. Furthermore, kinds of bacteria to survive in the intestine are different among individuals. It is necessary to select kinds of bacteria according to the intestinal environment for every individual. However, it is not easy. On the other hand, in the case of prebiotic preparation, in order to improve the intestinal bacterial flora, a large amount (5 - 10 g) of oligosaccharide needs to be orally administered, which makes administration difficult. Furthermore, when a necessary amount is administered at one dose, diarrhea and feeling of fullness may occur, which may worsen the symptoms of IBD.
[Non-patent document 1] K. L. Madsen et al: Lactobacillus species prevents colitis in interleukin 10-gene-deficient mice. Gastroenterology 116:1107-1114, 1999
[Patent document 1] Japanese Patent Application Unexamined Publication No. 2000-325045

### SUMMARY OF THE INVENTION

### [Problems to be Solved by the Invention]

An object of the present invention is to provide a composition effective in treatment or prevention of an inflammatory bowel disease (IBD).

### [Means to Solve the problem]

In order to achieve the above-mentioned object, the inventors of the present invention have made studies and reached a method for generating oligosaccharide in a living body by using an oligosaccharide generating enzyme i.e. glucosyltransferase, that is, a method for generating oligosaccharide in a living body from taken food by using the function of an oligosaccharide generating enzyme and activating proliferation of lactic acid bacteria originally surviving in the intestine of an individual with the use of the generated oligosaccharide. Then, on the assumption that the method is effective in treatment or prevention of IBD, the inventors carry out an experiment using an animal model. As a result, significant reduction of symptoms of IBD is observed in an oligosaccharide generating enzyme administered group. Thus, the efficacy of the method with respect to IBD is confirmed. Note here that the use of an oligosaccharide generating enzyme to improve the intestinal bacterial flora (and an enzyme composition to be used therefor) are described in Japanese Patent Application Unexamined Publication No. 2000-325045 (patent document 1) in detail. In the document, however, the application of an oligosaccharide generating enzyme to IBD is not suggested at all.
The present invention is based on the above-mentioned achievements and provides an enzyme composition, and the like, listed below.
[1] An enzyme composition for use in treating or preventing an inflammatory bowel disease, including an enzyme capable of generating oligosaccharide in a living body, wherein the enzyme is glucosyltransferase.
[2] The enzyme composition for use in according to [1], wherein the enzyme acts in a stomach.
[3] The enzyme composition for use in according to [1] or [2], wherein the enzyme catalyzes transglycosylation.
[4] The enzyme composition for use in according to any of [1] to [3], further comprising at least one enzyme selected from the group consisting of fructosyltransferase and levansucrase.
[5] The enzyme composition for use in according to any of [1] to [4], further including at least one enzyme selected from the group consisting of amylase and invertase.
[6] A food containing an enzyme composition according to any of [1] to [5]. A use of an enzyme capable of generating oligosaccharide in a living body for producing an enzyme composition for preventing or treating an inflammatory bowel disease, wherein the enzyme is glucosyltransferase.
[8] The use according to [7], wherein the enzyme acts in a stomach.
[9] The use according to [7] or [8], wherein the enzyme catalyzes transglycosylation.
[10] The use according to any of [7] to [9], wherein the enzyme is at least one enzyme selected from the group consisting of glucosyltransferase, fructosyltransferase and levansucrase.
[11] The use according to any of [7] to [10], wherein the enzyme composition is taken before, between or after meals.
[12] The use according to any of [7] to [10], wherein the enzyme composition is taken together with food.
[13] The use according to any of [7] to [12], wherein the enzyme composition further comprises at least one enzyme selected from the group consisting of amylase and invertase.

### BRIEF DESCRIPTION OF THE DRAWINGS

Fig. 1 shows a scheme of an experiment using IBD animal models.
Fig. 2 is a graph showing a change over time of body weights of a glucosyltransferase administered group and a non-administered group.
Fig. 3 is a graph showing a change over time of disease activity index (DAI) of a glucosyltransferase administered group and a non-administered group.
Fig. 4 is a graph showing comparisons of the number of anaerobic bacteria and the number of lactic acid bacteria in 1 g of feces between a glucosyltransferase administered group and a non-administered group (on Day 0 after administration of 3% DSS). (a) A graph showing a comparison of the number of anaerobic bacteria. (b) A graph showing a comparison of lactic acid bacteria (Bifidobacterium). CFU = colony forming unit
Fig. 5 is a graph showing comparisons of the number of anaerobic bacteria and the number of lactic acid bacteria in 1 g of feces between a glucosyltransferase administered group and a non-administered group (on Day 5 after administration of 3% DSS). (a) A graph showing a comparison of the number of anaerobic bacteria. (b) A graph showing a comparison of lactic acid bacteria (Bifidobacterium).
Fig. 6 shows histological examination of the large intestines of a glucosyltransferase administered group and a non-administered group. Left picture: normal tissue; center picture: a non-administered group (on Day 5 after administration of 3% DSS), and right picture: a glucosyltransferase administered group (on Day 5 after administration of 3% DSS).

### DETAILED DESCRIPTION OF THE INVENTION

A first aspect of the present invention relates to an enzyme composition for treating or preventing an inflammatory bowel disease (IBD). The enzyme composition of the present invention includes glucosyltransferase as an enzyme capable of generating oligosaccharide in a living body (hereinafter, also referred to as "oligosaccharide generating enzyme"). An enzyme acting in a living body by using a component in taken food as a substrate so as to generate oligosaccharide is used. Kinds of enzymes to be used are not particularly limited as long as the enzyme has the above-mentioned property. Furthermore, the origin of the enzyme is not limited, and any origins such as bacteria, fungi, yeast, actinomycetes, basidiomycetes, plants, or animals can be used. However, since the enzyme composition of the present invention exhibits the effect in a living body, an enzyme capable of acting in an environment in a living body is employed. In particular, it is preferable to use an enzyme capable of acting in the stomach stably and excellently (that is, an enzyme acting sufficiently even at low pH).

Kinds of oligosaccharides to be generated by the action of the enzyme composition of the present invention is not particularly limited. An example of the oligosaccharide may include fructo-oligosaccharides (those in which 1 to 3 fructose molecules are linked to the fructose residue of sucrose at the C2- and C1-positions by β-bonding), branched oligosaccharides (oligosaccharides having α-1,6 bond), galacto-oligosaccharides (such as raffinose and stachyose), gentio-oligosaccharides (oligosaccharides having β-1,6-glucoside bond), and the like. More specific examples may include 4-α-glucosyl-xylose, 3-α-glucosyl-sorbose, 4-α-glucosyl-sucrose, 4-α-glucosyl-mannose, 4-α-glucosyl-glucosamine, 4-α-glucosyl-N-acetyl-glucosamine, 3-,6-α-glucosyl-mannose, 3-,4-α-glucosyl-xylose, 1-,3-,4-α-glucosyl-fructose, α-glucosyl-glycerol, riboflavin-α-glucoside, 6-α-galactosyl-fructose, 6-α-galactosyl-galactose, α-galactosyl-glycerol, 3-,4-,6-β-galactosyl-lactose, β-galactosyl-glycerol, 4-β-galactosyl-glucose, 4-β-galactosyl-mannose, β-galactosyl-glycerol, xylosyl-fructoside, galactosyl-fructoside, isomaltosyl-fructoside, lactosyl-fructoside, 1-kestose, nistose (fructo-oligosaccharide), neokestose, inulobiose, difructofuranosyl 1,2':2,1':2,3':2,6':2'6-dianhydride, lactosucrose, and the like.
These hetero-oligosaccharides can be generated by using, for example, glucosyl transfer reaction, galactosyl transfer reaction, and fructosyl transfer reaction by glycosyltransferase. The glucosyl transfer reaction can use glucosyltransferase, cyclodextrin synthetase, amylomaltase, α-glucosidase, β-glucosidase, and the like. The galactosyl transfer reaction can use α-galactosidase, β-galactosidase, α-galactanase, and the like. The fructosyl transfer reaction can use fructosyltransferase, levansucrase, β-fructofuranosidase, cyclic disaccharide synthetase, and the like.

It is preferable that the enzyme composition of the present invention is formed by further using any one enzyme or two or more enzymes selected from fructosyltransferase, and levansucrase. Glucosyltransferase is an enzyme having a function of transferring glucose to generate isomaltose, panose and other oligosaccharides. Glucosyltransferase derived from microorganisms such as the genus Streptococcus, the genus Bacillus, the genus Aspergillus, the genus Aureobasidium and the genus Klebsiella, and plants such as onion are known. For example, glucosyltransferase derived from Aspergillus niger (trade name: Transglucosidase L "Amano" and α glucosidase "Amano" manufactured by Amano Enzyme Inc.) can be used for the enzyme composition of the present invention.

Fructosyltransferase is an enzyme that acts mainly on sucrose to cut α-1, β-1 bonds between fructose and glucose, and then to transfer fructose to sucrose so as to generate oligosaccharides. Its known origins include microorganisms such as the genus Bacillus, the genus Arthrobacter, the genus Aspergillus, the genus Fusarium, the genus Gloeosporium, the genus Saccharomyces, the genus Rhodotorula, the genus Pichia, the genus Hansenula and the genus Candida, asparagus and Jerusalem artichoke. For example, fructosyltransferase derived from Bacillus natto can be used for the enzyme composition of the present invention (Denpun Kagaku, vol. 38, No. 2, 217-222 (1991)).

Levansucrase is an enzyme that transfers fructose of sucrose to generate various oligosaccharides such as a high molecule polysaccharide levan. For example, levansucrase derived from Zymomonas mobilis IFO-13756 (Journal of Fermentation and Bioengineering, vol. 79, No. 4, 367-369 (1995)) and levansucrase derived from Rahnella aquatilis JCM-1683 can be used for the enzyme composition of the present invention.

To increase the kinds of substrates capable of acting and to improve the productivity of oligosaccharide from food, it is preferable to use a plurality of enzymes whose substrate-specificities are different from each other. Examples of such preferable enzyme compositions may include an enzyme composition containing glucosyltransferase and fructosyltransferase, an enzyme composition containing glucosyltransferase and levansucrase,
and an enzyme composition containing glucosyltransferase, fructosyltransferase and levansucrase.

Some kinds of foods to be taken may not include sufficient substrates of the oligosaccharide generating enzyme contained in the enzyme composition of the present invention. In such a case, it is desirable that a substrate of the oligosaccharide generating enzyme is formed from compositions of the food taken. One preferable embodiment of the enzyme composition of the present invention contains, in addition to the oligosaccharide generating enzyme, an enzyme such as amylase and invertase which is an enzyme that acts upon the taken food so as to produce a substrate of an oligosaccharide generating enzyme. Herein, amylase is an enzyme that acts on starch to generate disaccharides, trisaccharides and oligosaccharides that are substrates of the glycosyltransferase. For example, Aspergillus amylase derived from oryzae (trade name: Biodiastase 2000, manufactured by Amano Enzyme Inc.) can be used. On the other hand, invertase is an enzyme transferring sucrose into fructose and glucose. For example, invertase derived from S.cerevisiae can be used.

With problems of incompatibility taken into consideration, arbitrary components (for example, gastrointestinal preparation such as gastric antiacid and H₂ blocker) may be contained in the enzyme composition of the present invention.

The enzyme composition of the present invention can be formulated according to usual methods. In formulation, other pharmaceutically acceptable components (for example, carriers, vehicles, disintegrators, buffers, emulsifying agents, suspensions, stabilizers, preservatives, antiseptic agents, physiologic saline, and the like) can be contained. An example of the vehicle may include lactose, starch, sorbitol, D-mannitol, sucrose, and the like. An example of the disintegrator may include starch, carboxymethyl cellulose, calcium carbonate, and the like. An example of the buffers may include phosphate, citrate, acetate, and the like. An example of the emulsifying agents may include gum Arabic, sodium alginate, traganth, and the like. An example of the suspension may include glyceryl monostearate, aluminium monostearate, methyl cellulose, carboxymethyl cellulose, hydroxymethyl cellulose, sodium lauryl sulfate, and the like. An example of the stabilizer may include propylene glycol, diethyline sulfite, ascorbic acid, and the like. An example of the preservative may include phenol, benzalkonium chloride, benzyl alcohol, chlorobutanol, methylparaben, and the like. An example of the antiseptic agent may include benzalkonium chloride, parahydroxy benzoic acid, chlorobutanol, and the like.
Formulation forms are not particularly limited. A form of, for example, tablet, powder, fine granule, granule, capsule, syrup, and the like, may be employed.

The enzyme composition of the present invention is taken before, between, and after meals, or taken together with food. Since it is necessary and important to form a state in which the enzyme contained in the enzyme composition and a substrate component in food coexist in a living body, it is preferable that the enzyme composition of the present invention is taken before, after, or at the same time of meals.

In order to generate oligosaccharide from taken food efficiently, it is desirable that the enzyme composition of the present invention acts on food in the initial stage of digestion. From such a viewpoint, it is preferable that the enzyme composition of the present invention is formed by using an enzyme having a property that sufficiently acts under the acidic environment in the stomach. The above-mentioned glucosyltransferase derived from Aspergillus niger (trade name: Transglucosidase L "Amano" and α glucosidase "Amano," which are manufactured by Amano Enzyme Inc.), levansucrase derived from Zymomonas mobilis IFO-13756, and amylase derived from Aspergillus oryzae (trade name: Biodiastase 2000 manufactured by Amano Enzyme Inc.) are preferable components because they excellently act even in the acidic environment.

The "subject (patient)" to whom the enzyme composition of the present invention is applied is not particularly limited and includes human and non-human mammalians (including pet animals, domestic animals, and laboratory animals, and specifically including, for example, mouse, rat, guinea pig, hamster, monkey, cow, pig, goat, sheep, dog, cat, chicken, quail, and the like.). According to one preferable embodiment, the enzyme composition of the present invention is applied to human.

The administration amount (dosage amount) of the enzyme composition can be arbitrarily set on the condition that a function of generating oligosaccharide in a living body is exerted. The administration amount can be appropriately set considering the nature and purity of an enzyme to be used, or symptom, age, sex, body weight, and the like, of the subject (patient). A person skilled in the art can set an appropriate dosage amount while considering these factors. For example, in the case of the enzyme composition containing the above-mentioned glucosyltransferase, the administration amount of the enzyme composition is set so that the amount of glucosyltransferase becomes 10000 to 5000000 units/time. Similarly, in the case of the enzyme composition containing levansucrase, the administration amount is set so that the amount of levansucrase becomes 2 to 50000 units/time. In the case of the enzyme composition containing amylase, the administration amount is set so that the amount of amylase becomes 10 to 5000 units/time.
The administration schedule can be determined to once to five times a day, once every two days, or once every three days, and the like.

The present invention further provides food containing the above-mentioned enzyme composition. An example of the "food" in the present invention includes general foods (grains, vegetables, meat, various processed foods, sweets, soft drink, and alcoholic beverage, and the like), nutritional food supplement (supplement). The nutritional food supplement can be provided in a form of, for example, powder, granule, tablet, paste, and liquid. Food containing a component functioning as a substrate of the enzyme composition, or food such as cereal and furikake (condiments sprinkled on food) with which the component is taken together is included in the preferable example of the food in the present invention.

Note here that in this specification, unless otherwise specified, the enzymatic activities were measured by the following measurement method.

### <Glucosyltransferase activity>

α-methyl-D-glucoside is used as the substrate, and an enzyme solution is allowed to act thereon at 40°C and at pH 5.0. The amount of the enzyme generating 1 µg of glucose for 60 minutes is defined as one unit.

### <Levansucrase activity>

Measurement is carried out using F Kit (D-Glucose / D-Fructose) (manufactured by Boehringer-Mannheim GmbH). Sucrose is used as a substrate. The amount of the enzyme generating 1 mg/ml of glucose in the reaction solution is defined as one unit.

### <Amylase activity>

Measurement is carried out according to the method for testing starch saccharification activity (37°C and at pH 5.0), among the digestion activity testing methods described in The Pharmacopoeia of Japan (General Test Methods). The amount of the enzyme which increases the reduction power that is equivalent to 1 mg of glucose for one minute is defined as one unit.

### [EXAMPLE]

### 1. Obtaining of glucosyltransferase

Glucosyltransferase derived from Aspergillus niger (trade name: α glucosidase "Amano" manufactured by Amano Enzyme Inc.) was used. The glucosyltransferase activity of the enzyme used was 3,000 u/mg.

### 2. Administration of glucosyltransferase to IBD animal model

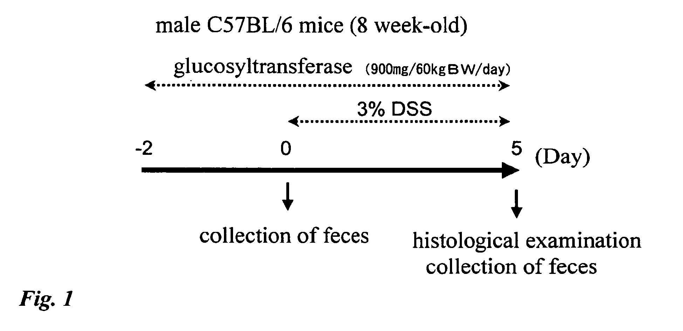

As an IBD animal model, a DSS enteritis model mouse (Sasaki M, et al. J Pharmacol Exp Ther. 2003; 305(1); 78-85) was used. This mouse is used as an IBD model since it has inflammation in the large intestine and presents IBD-like symptoms by administration of DSS (dextran sulfate sodium).
The experiment was carried out as follows. Eight (8) week-old male C57BL/6 mice (purchased from Chubu Kagaku Shizai Co., Ltd. (Nagoya)) were allowed to acclimate by keeping them for 7 days at 24°C, with access to food *ad libitum* and with an alternating 12 h light/dark cycle. After acclimation, mice were divided into two groups each composed of four mice. One group was allowed to orally take glucosyltransferase together with foods at a dosage of 900 mg (2,700,000 u) / 60 kg / day. Another group was allowed to orally take only food. As the food, a mixture of laboratory animal food MF (Oriental Yeast Co., Ltd.) and 10% (wt/wt) starch (Wako Pure Chemical Industries, Ltd.) was used. On Day 2 after the start of the oral administration of the enzyme, water containing 3% (wt/vol) dextran sulfate sodium (DSS, MW: 44 kDa, TdB Consultancy AB, Uppsala, Sweden) was continued to be given to both groups and bred for further five days (Fig. 1).

### 3. Verification of effect of suppressing body weight loss due to onset of IBD

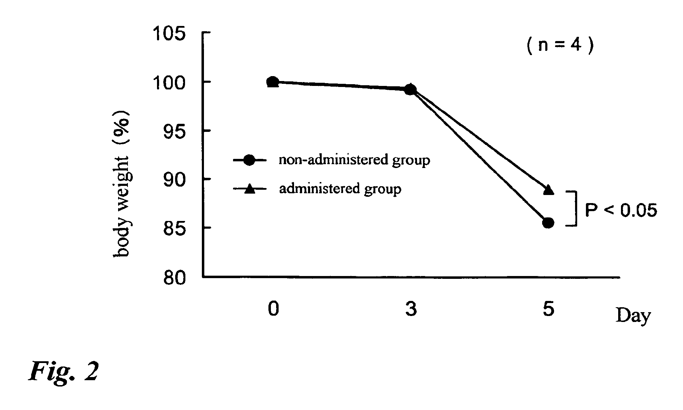

On Days 0, 3 and 5 after giving 3% DSS-containing water, body weights of mice of the glucosyltransferase administered group and those of the non-administered group were examined and compared with each other. In the mice of the non-administered group, body weight loss due to IBD was observed. On the other hand, in the glucosyltransferase administered group, the effect of suppressing the body weight loss was observed. On Day 5 after administration of 3% DSS-containing water, significant difference in suppression of the body weight loss was observed (Fig. 2). Thus, it is shown that the administration of glucosyltransferase brought the effect of suppressing the body weight loss due to IBD.

### 4. Verification of effect of preventing onset of IBD based on Disease activity index (DAI)

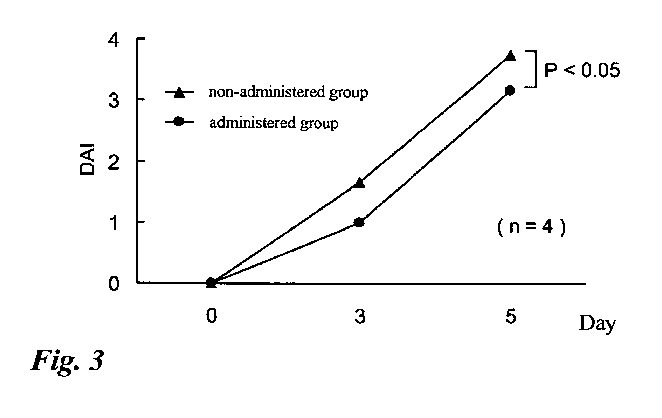

On Days 0, 3 and 5 after giving 3% DSS-containing water, the disease activity index (DAI) of the glucosyltransferase administered group and that of the non-administered group were measured and compared with each other. DAI is measured based on the body weight loss, state of feces, and bleeding states (Sasaki M, et al. J Pharmacol Exp Ther.2003; 305(1); 78-85). When the DAI of the glucosyltransferase administered group and the DAI of the non-administered group are compared with each other, a significant difference in the DAI is observed on Day 5 after the administration of 3% DSS-containing water (Fig. 3), obviously showing reduction of the DAI, that is, reduction of symptoms of IBD in the glucosyltransferase administered group.

### 5. Effect of improving intestinal bacterial flora

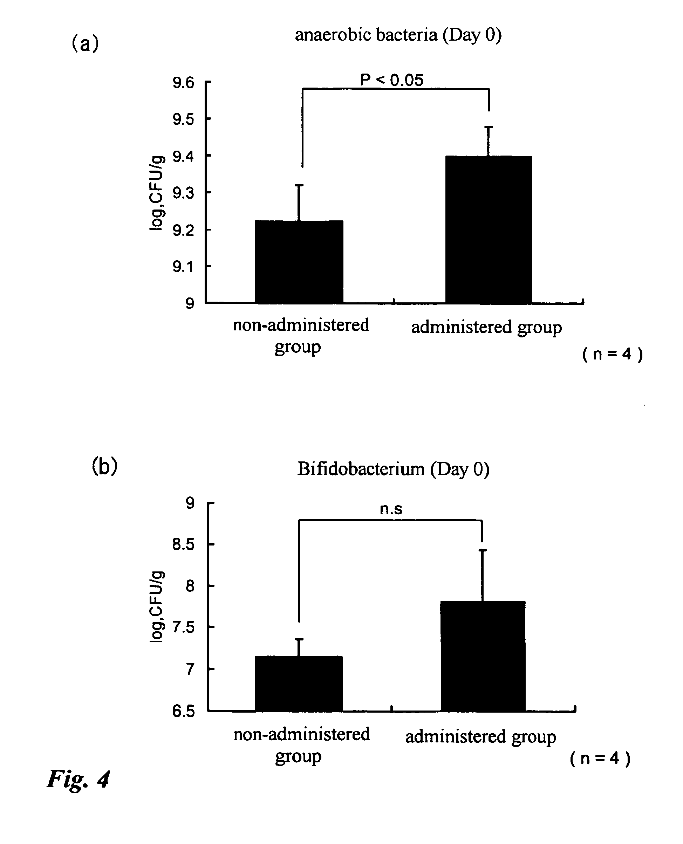

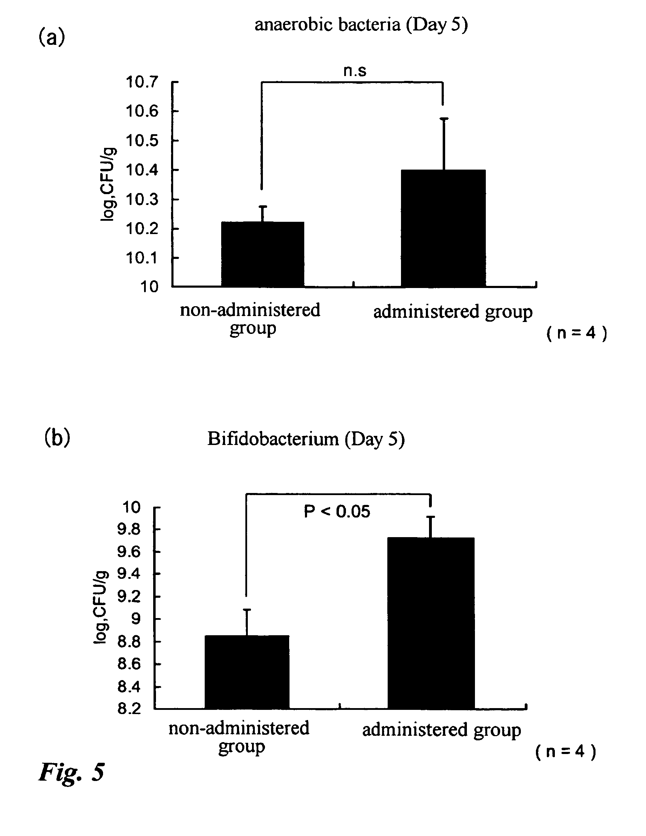

On Days 0 and 5 after administration of 3% DSS-containing water, feces of the glucosyltransferase administered group and the non-administered group were collected and then microorganisms in the feces were cultured. Then, the numbers of living bacteria of anaerobic bacteria and lactic acid bacteria such as Bifidobacterium in 1 g of feces were examined. On Day 0, the number of anaerobic bacteria in 1 g of feces in the glucosyltransferase administered group was significantly larger as compared with that of the non-administered group (Fig. 4 (a)). As to lactic acid bacteria such as Bifidobacterium, although a significant difference was not observed, the number of Bifidobacteria in 1 g of feces was larger in the glucosyltransferase administered group (Fig. 4 (b)).
Also on Day 5, the number of anaerobic bacteria in 1 g of feces in the glucosyltransferase administered group was larger although a significant difference was not observed (Fig. 5(a)). As to lactic acid bacteria such as Bifidobacterium, the number of Bifidobacteria in 1 g of feces was significantly larger in the glucosyltransferase administered group (Fig. 5(b)). These results show that the symptoms of IBD are reduced as a result of the increase of anaerobic bacteria and lactic acid bacteria such as bifidobacterium in the glucosyltransferase administered group.

### 6. Verification of IBD improvement effect from the viewpoint of anatomical findings

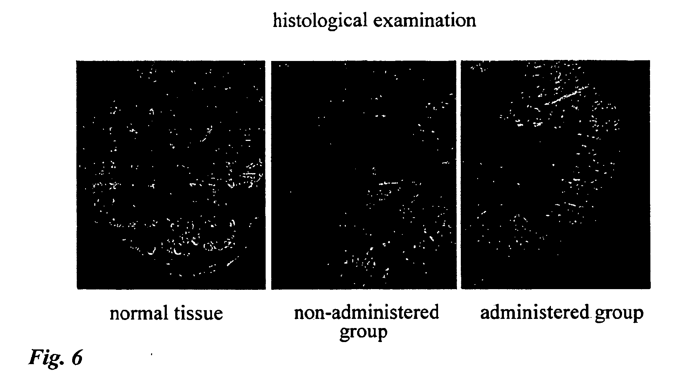

On Day 5 after administration of 3% DSS-containing water, the large intestines were extracted from the glucosyltransferase administered group and the non-administered group and subjected to histological examination. As a result, in the non-administered group, tissue destruction due to inflammation was observed (central picture of Fig. 6). In the glucosyltransferase administered group, it was observed that destruction of the tissue was suppressed (right picture of Fig. 6). Suppression of destruction of the endothelial tissue of the large intestine due to inflammation was observed in the glucosyltransferase administered group, showing suppression of the onset of IBD and reduction of symptoms by the administration of glucosyltransferase.

### [Industrial Applicability]

An enzyme composition of the present invention exhibits an effect of treating or preventing IBD through the improvement of intestinal bacterial flora. According to the enzyme composition of the present invention, it is not necessary to consider the survival of lactic acid bacteria unlike probiotic. Furthermore, as compared with the case of prebiotic, a necessary amount can be administered easily. Moreover, there is no concerns that an adverse effect due to overtake of oligosaccharide is triggered. In this way, the enzyme composition of the present invention has a large number of excellent properties as compared with conventional technologies. Much contribution of the enzyme composition to treatment or prevention of IBD is expected.

## Claims

1. An enzyme composition for use in treating or preventing an inflammatory bowel disease, comprising an enzyme capable of generating oligosaccharide in a living body, wherein the enzyme is glucosyltransferase.

2. The enzyme composition for use in according to claim 1, wherein the enzyme acts in a stomach.

3. The enzyme composition for use in according to claim 1 or 2, wherein the enzyme catalyzes transglycosylation.

4. The enzyme composition for use in according to any of claims 1 to 3, further comprising at least one enzyme selected from the group consisting of fructosyltransferase and levansucrase.

5. The enzyme composition for use in according to any of claims 1 to 4, further comprising at least one enzyme selected from the group consisting of amylase and invertase.

6. A food for use in treating or preventing an inflammatory bowel disease, containing an enzyme composition according to any of claims 1 to 5.

7. A use of an enzyme capable of generating oligosaccharide in a living body, for producing an enzyme composition for preventing or treating an inflammatory bowel disease, wherein the enzyme is glucosyltranferase.

8. The use according to claim 7, wherein the enzyme acts in a stomach.

9. The use according to claim 7 or 8, wherein the enzyme catalyzes transglycosylation.

10. The use according to any of claims 7 to 9, wherein the enzyme composition further comprises least one enzyme selected from the group consisting of fructosyltransferase and levansucrase.

11. The use according to any of claims 7 to 10, wherein the enzyme composition is designed to be taken before, between or after meals.

12. The use according to any of claims 7 to 10, wherein the enzyme composition is designed to be taken together with food.

13. The use according to any of claims 7 to 12, wherein the enzyme composition further comprises at least one enzyme selected from the group consisting of amylase and invertase.

## Patentansprüche

1. Enzymzusammensetzung zur Verwendung bei der Behandlung oder Vorbeugung einer entzündlichen Darmerkrankung, umfassend ein Enzym, das in der Lage ist Oligosaccharide in einem lebenden Körper zu generieren, wobei dieses Enzym Glucosyltransferase ist.

2. Enzymzusammensetzung zur Verwendung nach Anspruch 1, wobei das Enzym in einem Magen agiert.

3. Enzymzusammensetzung zur Verwendung nach Anspruch 1 oder 2, wobei das Enzym Transglycosylierung katalysiert.

4. Enzymzusammensetzung zur Verwendung nach einem der Ansprüche 1 bis 3, weiterhin umfassend zumindest ein Enzym ausgewählt aus der Gruppe bestehend aus Fruktosyltransferase und Levansucrase.

5. Enzymzusammensetzung zur Verwendung nach einem der Ansprüche 1 bis 4, weiterhin umfassend zumindest ein Enzym ausgewählt aus der Gruppe bestehend aus Amylase und Invertase.

6. Nahrungsmittol zur Verwendung bei der Behandlung oder Vorbeugung einer entzündlichen Darmerkrankung, umfassend eine Enzymzusammensetzung nach einem der Ansprüche 1 bis 5.

7. Verwendung eines Enzyms, das in der Lage ist Oligosaccharide in einem lebenden Körper zu generieren, zur Herstellung einer Enzymzusammensetzung zur Vorbeugung oder Behandlung einer entzündlichen Darmerkrankung, wobei das Enzym Glucosyltransferase ist.

8. Verwendung nach Anspruch 7, wobei das Enzym in einem Magen agiert.

9. Verwendung nach Anspruch 7 oder 8, wobei das Enzym Transglycosylierung katalysiert.

10. Verwendung nach einem der Ansprüche 7 bis 9, wobei die Enzymzusammensetzung weiterhin zumindest ein Enzym ausgewählt aus der Gruppe bestehend aus Fruktosyltransferase and Levansucrase umfasst.

11. Verwendung nach einem der Ansprüche 7 bis 10, wobei die Enzymzusammensetzung für die Einnahme vor, zwischen oder nach Mahlzeiten hergerichtet ist.

12. Verwendung nach einem der Ansprüche 7 bis 10, wobei die Enzymzusammensetzung für die Einnahme zusammen mit Nahrung hergerichtet ist.

13. Verwendung nach einem der Ansprüche 7 bis 12, wobei die Enzymzusammensetzung weiterhin zumindest ein Enzym umfasst ausgewählt aus der Gruppe bestehend aus Amylase und Invertase.

## Revendications

1. Composition enzymatique destinée à une utilisation dans le traitement ou la prévention de maladies inflammatoires chroniques de l'intestin, comprenant une enzyme pouvant générer un oligosaccharide chez un être vivant, dans laquelle l'enzyme est la glucosyltransférase.

2. Composition enzymatique destinée à une utilisation selon la revendication 1, dans laquelle l'enzyme agit dans l'estomac.

3. Composition enzymatique destinée à une utilisation selon la revendication 1 ou 2, dans laquelle l'enzyme catalyse une transglycosylation.

4. Composition enzymatique destinée à une utilisation selon l'une quelconque des revendications 1 à 3, comprenant en outre au moins une enzyme sélectionnée à partir du groupe constitué de la fructosyltransférase et de la lévane-sucrase,

5. Composition enzymatique destinée à une utilisation selon l'une quelconque des revendications 1 à 4, comprenant en outre au moins une enzyme sélectionnée à partir du groupe constitué de l'amylase et de l'invertase.

6. Aliment destiné à une utilisation dans le traitement ou la prévention de maladies inflammatoires chroniques de l'intestin, contenant une composition enzymatique selon l'une quelconque des revendications 1 à 5.

7. Utilisation d'une enzyme pouvant générer un oligosaccharide chez un être vivant, afin de produire une composition enzymatique destinée à empêcher ou à traiter des maladies inflammatoires chroniques de l'intestin, où l'enzyme est la glucosyltransférase.

8. Utilisation selon la revendication 7, où l'enzyme agit dans l'estomac.

9. Utilisation selon la revendication 7 ou 8, où l'enzyme catalyse une transglycosylation.

10. Utilisation selon l'une quelconque des revendications 7 à 9, où la composition enzymatique comprend en outre au moins une enzyme sélectionnée à partir du groupe constitué de la fructosyltransférase et de la lévane-sucrase.

11. Utilisation selon l'une quelconque des revendications 7 à 10, où la composition enzymatique est conçue ou réalisée pour être prise avant, entre ou après les repas.

12. Utilisation selon l'une quelconque des revendications 7 à 10, où la composition enzymatique est conçue ou réalisée pour être prise avec un aliment.

13. Utilisation selon l'une quelconque des revendications 7 à 12, où la composition enzymatique comprend en outre au moins une enzyme sélectionnée à partir du groupe constitué de l'amylase et de l'invertase.
